(19) Europäisches Patentamt
European Patent Office
Office européen des brevets

(11) Publication number: 0 389 389 A1

(12) **EUROPEAN PATENT APPLICATION**

(21) Application number: 90420130.8

(22) Date of filing: 09.03.90

(51) Int. Cl.5: C07D 301/10, C07D 303/04, C07D 303/32

(30) Priority: 20.03.89 US 325635

(43) Date of publication of application: 26.09.90 Bulletin 90/39

(84) Designated Contracting States: GR

(71) Applicant: EASTMAN KODAK COMPANY
343 State Street
Rochester, New York 14650(US)

(72) Inventor: Monnier, John Robert, c/o Eastman Kodak Company
Patent Department, 343 State Street
Rochester, New York 14650(US)
Inventor: Muehlbauer, Peter James, c/o Eastman Kodak Comp.
Patent Department, 343 State Street
Rochester, New York 14650(US)

(74) Representative: Parent, Yves et al
Kodak-Pathé Département Brevets et Licences Centre de Recherches et de Technologie Zone Industrielle
F-71102 Chalon-sur-Saône Cédex(FR)

(54) **Selective epoxidation of bicyclic olefins.**

(57) Process is disclosed for the selective epoxidation of bicyclic olefins. Such olefins are contacted with an oxygen-containing gas in the presence of a silver catalyst under defined reaction conditions, thereby selectively producing epoxides in good yield.

EP 0 389 389 A1

# Selective Epoxidation of Bicyclic Olefins

This invention relates to oxidation reactions. In one aspect, this invention relates to selective oxidation reactions for the production of epoxides from bicyclic olefins.

## Background of the Invention

Epoxides are highly reactive chemical compounds which, as a result of their reactivity, can be used in a wide variety of applications. Unfortunately, due to the reactivity of epoxides, they are often difficult to prepare with high selectivity and in high yields. Ethylene is the only olefin which has been successfully oxidized employing molecular oxygen on a commercial scale to produce an epoxide.

Preferred catalysts employed for the oxidation of ethylene to produce ethylene oxide comprise silver on solid supports. When such catalysts are employed for the oxidation of olefins having longer chain lengths than ethylene, no epoxides are obtained, but instead various higher oxidation products (up to and including carbon dioxide and water) are obtained.

Alternate routes to epoxides other than ethylene oxide include the non-catalytic oxidation of olefins with peroxides. Such processes are not only uneconomical, but are also hazardous due to the large quantities of peroxide required for the desired conversion.

It would, therefore, be desirable to be able to catalytically oxidize olefins other than ethylene to produce epoxides directly. Such processes would provide large quantities of highly reactive olefin derivatives which would find a wide range of uses, such as for example, as polymer cross-linking agents, as reactive chemical intermediates, as precursors for the production of organic solvents, and the like.

## Objects of the Invention

An object of the present invention, therefore, is to provide a catalytic process for the selective oxidation of bicyclic olefins to selectively produce epoxides in high yield.

This and other objects of the present invention will become apparent from inspection of the detailed description and appended claims which follow.

## Statement of the Invention

In accordance with the present invention, we have discovered that bicyclic olefins, i.e., olefins having no allylic hydrogens, or, when allylic hydrogens are present, such hydrogen atoms are present in such sterically hindered positions as to be essentially unreactive under reaction conditions, such olefins can be catalytically oxidized to produce a high selectivity of epoxide derivatives thereof by contacting the olefin feed with an oxygen-containing gas in the presence of a silver catalyst under defined oxidation conditions. The practice of the present invention makes possible the large scale production of such highly functionalized compounds as norbornene oxide, norbornadiene monoepoxide, bicyclo[2.2.2]oct-2-ene oxide, 5-ethyl-2-norbornene oxide and 5-norbornene-2-carboxaldehyde oxide employing readily available feedstocks (e.g., norbornene, norbornadiene, bicyclo[2.2.2]oct-2-ene, 5-ethyl-2-norbornene and 5-norbornene-2-carboxaldehyde, respectively). The only other material consumed during the invention reaction, besides the olefin feedstock, is molecular oxygen. Thus, the invention process is not only economical, but, since the reaction can be run in the continuous mode, it also makes possible the ready preparation of large quantities of these useful chemical compounds.

## Detailed Description of the Invention

In accordance with the present invention, we have developed a process for the selective epoxidation of bicyclic olefins having defined structure which comprises contacting the feed olefin with a sufficient quantity of an oxygen-containing gas so as to maintain the molar ratio of olefin to oxygen in the range of 0.01 up to 20, in the presence of a silver catalyst at a reaction pressure in the range of 0.1 up to 100 atmospheres and a temperature in the range of about 75 up to 325°C for a reaction time sufficient to obtain olefin

conversions per pass in the range of about 0.1 up to 75 mole percent.

Olefins contemplated for use in the practice of the present invention are [2.x.y] bicyclic olefins, i.e., olefins which satisfy the following structural formula

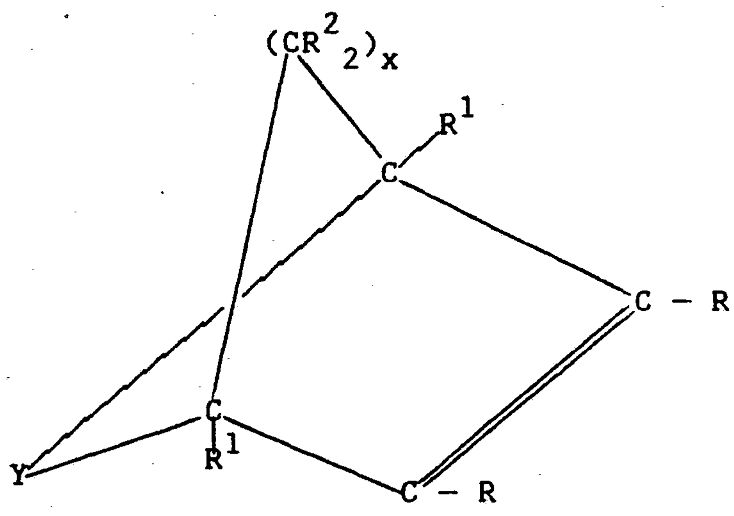

wherein

each R is independently selected from the group consisting of:

a) hydrogen,

b) aryl and substituted aryl groups having in the range of 6 up to 20 carbon atoms,

c) tertiary alkyl groups of the formula:

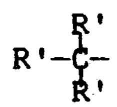
,

where each $R'$ is independently:

a $C_1$-$C_{10}$ alkyl or substituted alkyl group,

a $C_6$-$C_{20}$ aryl or substituted aryl group,

$CR''_3(CR''_2)_n-\overset{O}{\overset{\|}{C}}-$,

$CR''_3(CR''_2)_n-\overset{O}{\overset{\|}{C}}-O-$, or

$R''O-\overset{O}{\overset{\|}{C}}-$,

where $R''$ is H, a $C_1$-$C_{10}$ alkyl or substituted alkyl group, an aryl or substituted aryl group having 6 up to 20 carbon atoms, and n is a whole number from 0-12;

d) $CR''_3-(CR''_2)_x-O-$, where x is a whole number from 1-12;

e)

$CR''_3(CR''_2)_n-\overset{O}{\overset{\|}{C}}-$;

f)

$CR''_3(CR''_2)_n-\overset{O}{\overset{\|}{C}}-O-$;

g)

$R''O-\overset{O}{\overset{\|}{C}}-$;

h)

$R''_2N-\overset{O}{\overset{\|}{C}}-$;

i) $R''_2N-$;

j) $R''S-$;

k) $CR'_2=CR''\{CR''=CR''\}_y$, where y is an integer from 0-20; and

l) 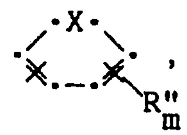

where X is O, S or $NR''$; and m is an integer from 0-3, with the proviso that the R groups shall have no allylic hydrogens;
each $R^1$ of said bicyclic olefin is H or a $C_1$-$C_{20}$ hydrocarbyl or substituted hydrocarbyl moiety;
each $R^2$ of said bicyclic olefin is H or a $C_1$-$C_{20}$ hydrocarbyl or substituted hydrocarbyl moiety;
x is a whole number which can vary from 1 up to 4;
Y is either an alkylene moiety of the structure:
$-(CR'_2)_y-$
wherein the $R'$ substituents are as defined above and each $R'$ is selected independently of the other, and y is a whole number which can vary from 1 up to 4. Alternatively, Y can be an alkenylene moiety of the structure:
$-\underset{R}{\overset{|}{C}}=\underset{R}{\overset{|}{C}}-$
wherein the R substituents are as defined above and each R is selected independently of the other. In such case, y for such alkenylene moiety is deemed to be 2.

Exemplary olefins which satisfy the above structural formula include norbornene, norbornadiene, bicyclo[2.2.2]oct-2-ene, 5-ethyl-2-norbornene, 5-norbornene-2-carboxaldehyde, and the like. A presently preferred olefin for use in the practice of the present invention is norbornene because of its ready availability, relatively low cost, and the wide range of possible uses for the epoxide reaction product.

The silver catalyst required for the practice of the present invention can be employed in either supported or unsupported forms.

When a support is employed, the loading level of silver on support typically falls within the range of about 0.1 up to 50 weight percent, calculated as elemental silver and based on the total weight of finished catalyst. Preferably, the loading level of silver on support falls within the range of about 1 up to 30 weight percent elemental silver; with loading levels in the range of about 2 up to 20 weight percent being most preferred.

It is presently preferred to apply the silver to a solid support for efficient use of the expensive silver component. Typical catalyst supports include
silica,
alumina,
silica-alumina,
titanium oxide,
lanthanum oxide,
magnesium oxide,
boron nitride,
boron carbide,
silicon nitride,
silicon carbide,
zinc oxide,
tin oxide,
iron oxide,
calcium oxide,
barium oxide,
strontium oxide,
zirconium oxide,
carbon,
boron phosphate,
zirconium phosphate,
and the like, as well as mixtures of any two or more thereof.

Typically, these solid supports will have a surface area of less than about 50 $m^2/g$. Preferred supports will have a surface area of less than about 10 $m^2/g$ and will be neutral or moderately basic in character. The

presently most preferred supports have surface areas of less than about 1 $m^2/g$, and include alumina, silica, and silicon carbide.

The actual physical form of the catalyst support is not particularly important. While the form of the catalyst support has little effect on catalyst activity, practical considerations such as ease of heat transfer, mass transfer, pressure drop due to fluid flow restrictions, efficiency of gas-liquid-solid contacting, catalyst durability, and the like make the use of defined shapes such as spheres, pellets, extrudates, rings, saddles, and the like preferred.

Especially preferred supports are those which have been treated with in the range of 0.001 up to 10 weight %, based on the total weight of catalyst, including support, of at least one promoter selected from the group consisting of:
the salts of alkali metals,
the oxides of alkali metals,
the salts of alkaline earth metals,
the oxides of alkaline earth metals,
organic halides,
inorganic halides,
acid halides, and
elemental halogens. Exemplary salts of alkali metals include sodium nitrate, sodium sulfate, sodium chloride, sodium bromide, rubidium nitrate, rubidium acetate, lithium sulfate, lithium chloride, cesium nitrate, cesium chloride, cesium bromide, and the like; exemplary oxides of alkali metals include sodium oxide, sodium hydroxide, cesium oxide, cesium hydroxide, lithium oxide, lithium hydroxide, and the like; exemplary salts of alkaline earth metals include barium nitrate, barium acetate, calcium nitrate, calcium acetate, calcium chloride, and the like; exemplary oxides of alkaline earth metals include barium oxide, barium hydroxide, calcium oxide, calcium hydroxide, and the like; exemplary organic halides include chloroform, bromoform, methylene chloride, methylene bromide, ethylene dibromide, ethylene dichloride, dichloroethane, dibromoethane, dichloropropane, vinyl chloride, chlorobenzene, bromobenzene, α-chlorotoluene, 2-chlorotoluene, and the like; exemplary inorganic halides include HCl, HBr, and the like; exemplary acid halides include HOCl, HOBr and the like; and the elemental halogens include chlorine, bromine and iodine. Those of skill in the art recognize that the above-recited compounds are merely illustrative of the compounds which are useful as promoters in the practice of the present invention, and that many other compounds which fall within the generic categories set forth above can also be identified and would be expected to also impart enhanced activity and/or selectivity to the catalyst employed in the practice of the present invention.

Of the above compounds, the alkali metal halides and alkali metal nitrates are presently most preferred. Exemplary preferred alkali metal halides include cesium chloride, rubidium chloride, potassium chloride, sodium chloride, sodium bromide, potassium bromide, rubidium bromide, cesium bromide, and the like. Exemplary preferred alkali metal nitrates include cesium nitrate, rubidium nitrate and potassium nitrate.

Those of skill in the art recognize that catalysts employed in the practice of the present invention can include additional components which may modify catalyst activity and/or selectivity. Such additives may be incorporated into the finished catalyst because their presence aids catalyst preparation, e.g., binders, die lubricants, and the like; or additives may be incorporated as extenders to reduce the cost of catalyst preparation; or additives may be incorporated to extend the operating ranges for reaction temperature and/or pressure; or additives may be incorporated to increase catalyst lifetime under reaction conditions and/or to modify the amounts of catalyst promoters employed to produce enhanced catalyst activity. It is recognized, of course, that some additives (e.g., cesium) are suitably employed in very low levels (i.e., milligrams of additive per gram of catalyst); while other additives (i.e., binders, diluents, and the like) are suitably employed at significantly higher levels (i.e., as a significant percentage of the total catalyst weight).

Supported catalysts can be prepared employing techniques well known to those of skill in the art, such as, for example, by precipitation of the active metals on the support, by impregnation, by coprecipitation of support and active metals, by grinding together solid support and active metal(s) in particulate form; and the like. When a promoter is also present in the catalyst, the order in which it is incorporated into the catalyst is not critical, i.e., support can be contacted with a silver source, then promoter; or support can be contacted with promoter, then a silver source; or support can be contacted simultaneously with both promoter and a silver source; and other such variations.

Most any source of silver is suitable for use in preparing the catalyst employed in the practice of the present invention. Since a preferred method for preparation of supported catalyst involves impregnation of support with a solution of a silver compound, soluble silver compounds are a presently preferred source of silver. Exemplary compounds are silver nitrate, silver oxalate, silver acetate, and the like. Those of skill in

the art recognize that certain organic silver compounds require the addition of ammonia or an amine in order to solubilize the organic silver compound in aqueous medium; thus, the use of such solvation-promoting additives is contemplated in the practice of the present invention.

The process of the present invention is carried out under oxidation conditions, i.e., in the presence of sufficient quantities of an oxygen-containing gas to provide a molar ratio of olefin to oxygen in the range of about 0.01 up to 20. While greater or lesser quantities of molecular oxygen can be employed, sufficient quantities of oxygen should be provided to insure that undesirably low levels of olefin conversion do not occur, while excessively high oxygen concentrations should be avoided to prevent the formation of explosive mixtures.

Suitable oxygen-containing gases include air, oxygen-enriched air, substantially purified oxygen, oxygen diluted with inert gases such as $N_2$, Ar, $CO_2$ or $CH_4$, and the like.

Optionally, the process of the present invention can be carried out by contacting the olefin to be oxidized with molecular oxygen and about 0.1 up to 1000 parts per million (by volume of total feed) of organic halide. Lower levels of organic halide will provide negligible effect on catalyst performance, while higher levels of organic halide would not be expected to provide any significant improvement in catalyst performance.

Preferred quantities of organic halide for use in the practice of this optional embodiment of the present invention fall within the range of about 1 up to 100 parts per million, by volume of total feed.

Organic halides contemplated for use in the practice of this optional embodiment of the present invention include compounds of the structure $R^3X$, wherein $R^3$ is hydrocarbyl radical or halogenated hydrocarbyl radical having in the range of 1 up to 8 carbon atoms, and X is any one of the halogens, preferably chlorine or bromine, and wherein $R^3$ contains at least one hydrogen atom which is sufficiently acidic so as to render $R^3X$ capable of undergoing dehydrohalogenation under the reaction conditions. Exemplary organic halides include $C_1$ compounds such as methyl chloride, methyl bromide, methylene chloride, methylene bromide, chloroform and bromoform, and the like; $C_2$ compounds such as ethyl chloride, ethyl bromide, dichloroethane, dibromoethane, vinyl chloride, dichloroethylene, trichloroethylene, and the like; $C_3$ compounds such as dichloropropane, dibromopropane, dichloropropene, dibromopropene, and the like; $C_4$ compounds such as chlorobutane, bromobutane, dichlorobutane, dibromobutane, chlorobutene, bromobutene, dichlorobutene, dibromobutene, and the like; $C_5$ compounds such as mono-, di-, tri-, tetra- and pentachloropentanes or pentenes, mono-, di-, tri-, tetra- and pentabromopentanes or pentenes, cyclopentylchloride, cyclopentylbromide, and the like; $C_6$ compounds such as mono-, di-, tri-, tetra-, penta- and hexachlorohexanes or hexenes, mono-, di-, tri-, tetra, penta-and hexabromohexanes or hexenes, cyclohexylchloride, cyclohexylbromide, chlorobenzene, bromobenzene, and the like; $C_7$ compounds such as chlorotoluene, bromotoluene, benzylchloride, benzyl bromide, mono-, di-, tri-, tetra-, penta-, hexa- and heptachloroheptanes or heptenes, mono-, di-, tri-, tetra-, penta- hexa-, hepta- and octachlorooctanes or octenes, mono-, di-, tri-, tetra-, penta-, hexa-, hepta-, and octabromooctanes or octenes, and the like; as well as mixtures of any two or more thereof.

The organic halide, when used, can be added to the oxidation reaction zone in a variety of ways. For example, it can be mixed with the olefin to be oxidized and/or the oxygen-containing gas prior to contacting with the catalyst, or the organic halide can be introduced to the reaction zone separately from the feed olefin and/or the oxygen-containing gas.

Suitable reaction temperatures fall within the range of about 75 up to 325 °C. At lower temperatures, the reaction proceeds so slowly as to be impractical, while at higher temperatures undesirable levels of byproducts, e.g., carbon dioxide, are obtained. Preferred reaction temperatures fall within the range of about 125 up to 300 °C; with temperatures in the range of about 175 up to 275 °C being most preferred because selectivity to the desired monoepoxide falls off at temperatures significantly above 275 °C and space-time yields are undesirably low at temperatures below about 175 °C.

The reaction pressure can vary within wide ranges, with typical limits of about 0.1 up to 100 atmospheres being chosen primarily as a function of safety, handling, equipment and other practical considerations. Preferably, reaction pressure is maintained in the range of about 1 up to 30 atmospheres.

Reaction times suitable for the practice of the present invention can vary within wide ranges. Generally, olefin, oxygen and catalyst are maintained in contact for a time sufficient to obtain olefin conversions in the range of about 0.1 up to 75 mole percent. Reaction times sufficient to obtain olefin conversion in the range of about 2 up to 50 mole percent are preferred for efficient utilization of the reactor capacity.

Those of skill in the art recognize that the actual contact times required to accomplish the desired conversion levels can vary within wide ranges, depending on such factors as vessel size, olefin to oxygen ratios, the silver loading level on the catalyst, the presence or absence of any catalyst modifiers (and their loading levels), the reaction temperature and pressure, and the like.

The invention process can be carried out in either batch or continuous mode. Continuous reaction is presently preferred since high reactor throughput and high purity product is obtained in this manner. The batch mode is satisfactorily employed when high volume of reactant throughput is not required, for example, for liquid phase reactions.

For continuous mode of reaction carried out in the gas phase, typical gas hourly space velocities (GHSV) fall within the range of about 100 up to 30,000 $hr^{-1}$. GHSV in the range of about 200 up to 20,000 $hr^{-1}$ are preferred, with GHSV in the range of about 300 up to 8000 $hr^{-1}$ being most preferred because under such conditions the most desirable combination of feed olefin conversion and product selectivities are obtained.

When continuous mode of reaction is carried out in the liquid phase, typical liquid hourly space velocities (LHSV) employed will give contact times analogous to that obtained at the GHSV values given above. Most preferably, LHSV employed will fall in a range so as to produce the most desirable combination of feed olefin conversion levels and high product selectivity.

Recovery of product produced in the practice of the present invention can readily be carried out employing techniques well known by those of skill in the art. For example, where reaction is carried out in the continuous mode, unreacted starting material is initially separated from reaction products; and the desired product then isolated from the resulting product mixture by distillation, crystallization, extraction, or the like. Since the selectivity to the desired epoxide product is generally quite high, there are only small amounts of undesired reaction products from which to isolate the desired product.

The invention will now be described in greater detail by reference to the following non-limiting examples.

EXAMPLE 1 - Catalyst Preparation

Catalysts were typically prepared by impregnation of support with a solution of a silver compound (and optionally a promoter) in 0.5-2 volumes of solvent relative to the volume of support being treated.

For example, a 17% Ag (as determined by neutron activation analysis) on $Al_2O_3$ support was prepared by dissolving 38 grams of Kodak silver nitrate in 100 mL of distilled water. One hundred grams of fluidizable alumina powder (surface area 0.26 $m^2/g$, total pore volume 0.675 cc(Hg)/gm, median pore diameter 19$\mu$, packing density 0.98 $g/cm^3$, chemical composition (wt %): $Al_2O_3$ - 84.7, $SiO_2$ - 13.4, $Fe_2O_3$ - 0.21, $TiO_2$ - 0.47, CaO - 0.21, MgO - 0.12, $Na_2O$ - 0.15, $K_2O$ - 0.26) were added to the silver-containing solution, the mixture tumbled for 30 minutes at 50° C, then water removed under vacuum at 60° C. The resulting powder was then dried for 60 minutes at 150° C in a forced air oven. This material, designated as Catalyst A, could be calcined and used directly for oxidation of olefin feed or treated with a promoter and then calcined.

Prior to catalyst evaluation (and either before or after further treatment with promoter), all catalysts were optionally calcined in an oxygen-containing atmosphere (air or oxygen-supplemented helium) at about 350° C for about 4 hours. Following calcination, all catalysts were subjected to an activation treatment at a temperature in the range of about 300-350° C in an atmosphere initially containing about 2-5% hydrogen in an inert carrier such as helium or nitrogen. The hydrogen content of the activating atmosphere was gradually increased up to a final hydrogen concentration of about 20-25% at a controlled rate so that the activation temperature did not exceed 350° C. After the temperature was maintained for about 1 hour at a hydrogen concentration in the range of about 20-25%, catalyst was ready for use.

When the $Ag/Al_2O_3$ catalyst was treated with promoter, a quantity of catalyst was contacted with 0.5-2 volumes of aqueous promoter, then dried as previously described. In this way, Catalysts B-H (details in Table 1) were prepared. Unless otherwise noted in the Table, catalyst support employed was the same fluidizable alumina powder as described above.

Silver-containing catalysts were also prepared using 1/4 inch diameter alumina rings. Thus, five hundred grams of 1/4 inch alumina rings having a surface area of 0.43 $m^2/g$, a total pore volume of 0.37 cc (Hg)/gm, a median pore diameter of 7$\mu$, a packing density of 0.80 $g/cm^3$, and a chemical composition (wt %) as follows: $Al_2O_3$ - 93.1, $SiO_2$ - 5.6, $Fe_2O_3$ - 0.3, $TiO_2$ - 0.1, CaO - 0.1, MgO - 0.3, $Na_2O$ - 0.1, $K_2O$ - 0.1 were added to a solution of 202 g of silver nitrate (Kodak) in 500 mL of distilled water. The resulting mixture was tumbled for 30 minutes at about 50° C, then the water was removed on a rotary evaporator under reduced pressure at about 60° C. The silver nitrate impregnated rings were then further dried for 30 minutes at about 120° C in a forced air oven. The dried material was then calcined for 4 hours in flowing air at about 350° C. After calcination, this ring-supported catalyst was promoted with 696 mg of CsCl dissolved in 500 mL of distilled water. The mixture was "pre-dried", i.e., under reduced pressure at about 60° C, and further dried in the forced air oven as described above. The resulting catalyst is designated as Catalyst I. A variation of

Catalyst I was prepared by similar promoter addition to a 12% silver loading-alumina ring catalyst. Material designated as Catalyst J was prepared from 200 g of 12% Ag, 224 mg of CsCl and 32 mg of $CsNO_3$.

Employing the same general procedure as set forth above, supported silver catalysts were prepared employing as support materials other than alumina. Thus, 15.4% Ag on ZnO was prepared by dissolving 9.5 g of $AgNO_3$ in 75 mL of distilled water, then adding to the solution 25 g of uncalcined ZnO (having a surface area of 3.9 $m^2$/gram, and a particle diameter in the range of about 75-150μ). This material was then dried and calcined as described above. The dried material was then treated with promoter as previously described employing 1.0 mg of $CsNO_3$ per gram of supported catalyst. The resulting catalyst is designated as Catalyst K. A variation of Catalyst K was prepared by treating the 15.4% Ag/ZnO catalyst with 1.0 mg of CsCl per gram of supported catalyst. The resulting material is designated as Catalyst L.

Titania supported catalyst was prepared by calcining $TiO_2$ (having a surface area of about 0.5 $m^2$/g, a particle diameter in the range of about 40-75μ) in oxygen at 450° for about 4 hours. Twenty five (25) grams of this treated $TiO_2$ was then slurried in about 50 mL of a distilled water solution containing 9.5 grams of $AgNO_3$. The combination was thoroughly mixed, then dried as described above. The resulting catalyst contained about 18% Ag. This material was then promoted with 1.0 mg of CsCl per gram of catalyst and is designated as Catalyst M.

Table 1

Supported Silver Catalysts

| Catalyst | Silver loading, wt % | Support | Promoter loading, mg/g* (Promoter Source) |
|---|---|---|---|
| A | 17 | $Al_2O_3$ powder | 0 |
| B | 17 | $Al_2O_3$ powder | 1.0 ($CsNO_3$) |
| C | 17 | $Al_2O_3$ powder | 1.0 (CsCl) |
| D | 17 | $Al_2O_3$ powder | 3.0 ($RbNO_3$) |
| E | 17 | $Al_2O_3$ powder | 1.3 (CsBr) |
| F | 17 | $Al_2O_3$ powder | 3.0 (RbCl) |
| G | 25 | $Al_2O_3$ powder | 1.0 ($CsNO_3$) |
| H | 5 | $Al_2O_3$ powder | 1.0 (CsCl) |
| I | 15 | $Al_2O_3$ rings | 1.0 (CsCl) |
| J | 12 | $Al_2O_3$ rings | 1.12 (CsCl)<br>0.16 ($CsNO_3$) |
| K | 15 | ZnO | 1.0 ($CsNO_3$) |
| L | 15 | ZnO | 1.0 (CsCl) |
| M | 18 | $TiO_2$ | 1.0 (CsCl) |

*Milligrams of promoter (based on weight of promoter compound) per gram of supported silver catalyst.

EXAMPLE 2 - Effect of Silver Loading Level

In all of the following catalyst evaluation runs, catalysts were evaluated under steady state conditions in a 1 atmosphere, single-pass flow reactor system.

The reactor tube was constructed of pyrex and the catalyst charge (between 0.1 and 20.0 g) was held in place by means of a pyrex frit. The geometries of the reactor and catalyst particles as well as bed depth were chosen to maintain and measure the true kinetic and catalytic aspects of the reaction. A chromel/alumel thermocouple sheathed in stainless steel was embedded within the catalyst bed to measure the true reaction temperature.

The feed gas $O_2$, as well as the diluent He, were added using mass flow controllers, which permitted highly accurate and reproducible flow rates of $O_2$ and He regardless of pressure changes from the supply cylinders or the reactor system downstream from the controllers. The bicyclic olefin feed was introduced into the reactor by passing the He diluent flow through a vapor saturator (containing the bicyclic olefin

material). The vapor saturator was maintained at a constant temperature so as to ensure that a reproducible concentration of bicyclic olefin is maintained in the feed stream. Typically, the saturator temperature was varied between about 0 up to 100° C, as a function of the bicyclic olefin being used, and as a function of the desired concentration of bicyclic olefin in the feed stream.

Reaction product analyses (as well as feed composition analyses) were made using an in-line gas sampling loop connected directly to the inlet of a Varian 3760 gas chromotograph. Both thermal conductivity (TC) and flame ionization (FI) detectors [(connected in series below the packed Chromosorb 101 column (8 ft. by 2mm id pyrex capillary column)] were used to analyze all of the reaction products. The TC detector gave quantitative analyses for $O_2$, $CO_2$, $H_2O$, and HCHO (if present), while the FI detector was used for organic molecules such as norbornene and norbornene oxide. In practice usually the only organic molecules present are the selective epoxidation product and olefin feedstock. By means of a switching valve, it was possible to divert the feed stream through the in-line sample loop prior to passage over the catalyst. In this way, quantitative analysis of the feed stream and comparison to the corresponding data from the reactor effluent were possible, thereby providing very accurate measurements of both conversion levels and product selectivities. Output from both the TC and FI detectors were integrated using computing integrators which were programmed to give both absolute quantities and rates of formation. All reactor exit lines were heated and maintained at 125-140° C to prevent product condensation.

The GC analysis was performed using the following temperature programming schedule: an initial temperature of 100° C was held for 2 minutes, followed by a temperature program rate of +10° C/min up to a final temperature of 200° C which was then held for 10 minutes. The helium GC carrier rate was 20 mL/min.

In this example, the effect of silver loading level on feed olefin conversion and product selectivity was investigated. Reaction parameters and results are presented in Table 2.

Table 2

| Reaction Parameters | | | | | |
|---|---|---|---|---|---|
| Catalyst | Temp. °C | GHSV, $hr^{-1}$ | Gas Feed*** ($He/Nor/O_2$) | Product | |
| | | | | Norbornene Conversion, % | Selectivity,**** % |
| H (5% Ag on $Al_2O_3$)* | 225 | 900 | 0.64:0.03:0.33 | 21 | 94 |
| | 250 | 900 | 0.64:0.03:0.33 | 26 | 90 |
| C (17% Ag on $Al_2O_3$)* | 200 | 400 | 0.64:0.03:0.33 | 4 | 93 |
| | 225 | 800 | 0.65:0.02:0.33 | 9 | 84 |
| B (17% Ag on $Al_2O_3$)** | 200 | 720 | 0.83:0.01:0.16 | 26 | 83 |
| | 217 | 720 | 0.66:0.01:0.33 | 59 | 71 |
| G (25% Ag on $Al_2O_3$)** | 225 | 1200 | 0.45:0.05:0.50 | 25 | 93 |
| | 250 | 1800 | 0.64:0.03:0.33 | 51 | 85 |

*CsCl promoted (1.0 mg CsCl/g catalyst)
**$CsNO_3$ promoted (1.0 mg $CsNO_3$/g catalyst)
***Volumetric ratio of feed components
****Product selectivity is reported as molar selectivity to norbornene epoxide based on the moles of norbornene converted. Carbon dioxide is the only other carbon-based product formed.

The above results demonstrate that high catalyst activity and selectivity are obtained with supported silver catalysts over a wide range of loading levels. As one might expect, higher activities are obtained at higher silver loading levels. It is of note that selectivity is not adversely affected even at increasing norbornene conversion levels.

EXAMPLE 3 - Effect of Adding Promoters

A series of catalyst evaluations were carried out employing the same experimental setup described in

Example 2. A variety of promoted catalysts were tested, with reaction parameters and results set forth in Table 3.

Table 3

| Reaction Parameters | | | | | |
|---|---|---|---|---|---|
| Effect of Promoters on Catalytic Activity of $Ag/Al_2O_3$ Catalysts | | | | | |
| Catalyst; Promoter loading, mg/g* (source) | Temp. °C | GHSV, $hr^{-1}$ | Gas Feed** (He/Nor/$O_2$) | Norbornene Conversion, % | Product Selectivity,*** % |
| A; O | 225 | 720 | 0.64:0.03:0.33 | 1 | 8 |
| | 250 | 720 | 0.64:0.03:0.33 | 9 | 3 |
| B****; 1.0 ($CsNO_3$) | 200 | 700 | 0.65:0.02:0.33 | 21 | 90 |
| | 214 | 700 | 0.65:0.02:0.33 | 40 | 86 |
| | 200 | 700 | 0.83:0.01:0.16 | 26 | 83 |
| | 225 | 700 | 0.65:0.02:0.33 | 48 | 86 |
| | 225 | 700 | 0.44:0.06:0.50 | 28 | 90 |
| C****; 1.0 (CsCl) | 200 | 400 | 0.64:0.03:0.33 | 4 | 93 |
| D****; 3.0 ($RbNO_3$) | 225 | 660 | 0.64:0.03:0.33 | 14 | 86 |
| | 250 | 660 | 0.64:0.03:0.33 | 24 | 77 |
| E****; 1.3 (CsBr) | 225 | 600 | 0.64:0.03:0.33 | 10 | 92 |
| | 250 | 600 | 0.64:0.03:0.33 | 18 | 91 |
| F****; 3.0 (RbCl) | 225 | 660 | 0.64:0.03:0.33 | 3 | 78 |
| | 225 | 660 | 0.80:0.03:0.17 | 11 | 56 |
| I*****; 1.0 (CsCl) | 225 | 220 | 0.64:0.03:0.33 | 46 | 83 |
| | 225 | 440 | 0.81:0.02:0.17 | 43 | 86 |
| | 250 | 440 | 0.81:0.02:0.17 | 52 | 77 |
| J******; 1.12 (CsCl), 0.16 ($CsNO_3$) | 225 | 270 | 0.62:0.05:0.33 | 44 | 89 |

*Milligrams of promoter (based on weight of promoter compound) per gram supported silver catalyst
**Volumetric ratio of feed components
***Product selectivity is defined as molar % selectivity to norbornene oxide based on the moles of nobornene converted. Carbon dioxide is the only other carbon-based product formed during reaction.
****Unpromoted form of this catalyst is Catalyst A.
*****Unpromoted catalyst is 15% Ag on $Al_2O_3$ rings.
******Unpromoted catalyst is 12% Ag on $Al_2O_3$ rings.

These results demonstrate that alkali metal compounds increase the rate of norbornene oxide formation and/or increase the selectivity to the desired product (relative to that obtained with unpromoted catalyst), with both rate and selectivity frequently being improved. The addition of halogens, in this case as alkali metal halides, is also shown to be an effective means to increase the selectivity to norbornene oxide.

The benefit of these additives is observed to be independent of the silver loading and the nature of the support employed.

EXAMPLE 4 - Effect of Various Catalyst Supports

A series of catalyst evaluations were carried out employing the same experimental setup described in Example 2. Three additional supported catalysts (Catalysts K, L and M), prepared using different catalyst supports, were tested. The reaction parameters and results are set forth in Table 4. For comparison, data are included in Table 4 for reaction with a similar catalyst on alumina support. In addition, data for a CsCl-promoted sample of unsupported silver powder are included. The latter material, designated as Catalyst N, was made by contacting 5.0 grams of 100 mesh silver powder (99.9999% purity) with an aqueous $CsNO_3$ solution employing the procedure described above. The finished catalyst contained 1.0 mg of $CsNO_3$ per gram of silver powder.

Table 4

| Effect of Support on Catalytic Activity | | | | | | |
|---|---|---|---|---|---|---|
| Reaction Parameters | | | | | | |
| Catalyst* | Support | Temp. °C | GHSV, $hr^{-1}$ | Gas Feed ($He/Nor/O_2$)** | Norbornene Conversion, % | Product Selectivity,*** % |
| N | None | 250 | 800 | 0.64:0.03:0.33 | 0.2 | ≦100**** |
| B | $Al_2O_3$ | 200 | 720 | 0.83:0.01:0.16 | 26 | 83 |
| | | 217 | 720 | 0.66:0.01:0.33 | 59 | 71 |
| K | ZnO | 225 | 700 | 0.64:0.03:0.33 | 59 | 82 |
| L | ZnO | 225 | 860 | 0.64:0.03:0.33 | 48 | 86 |
| | | 225 | 2840 | 0.89:0.01:0.10 | 56 | 88 |
| M | $TiO_2$ | 225 | 1050 | 0.64:0.03:0.33 | 28 | 90 |
| | | 250 | 1050 | 0.64:0.03:0.33 | 42 | 83 |
| | | 250 | 2800 | 0.87:0.01:0.12 | 35 | 84 |

*For amount and source of promoter loading, see Table 1.
**Volumetric ratio of feed components
***Product selectivity is the molar selectivity to norbornene oxide, based on the moles of norbornene converted. Carbon dioxide was the only other carbon-based product formed during the reaction.
****Because of the low conversion level, the amount of carbon dioxide formed was too small to quantitatively determine.

These results demonstrate that a variety of supports are effective for the highly selective conversion of norbornene to norbornene oxide. The results also indicate that while unsupported silver catalysts are operable for the practice of the present invention, the low rate of conversion and high catalyst cost would generally make such unsupported catalysts impractical for widespread use. These results also indicate that alumina is a useful support for the practice of the present invention.

EXAMPLE 5- Selective Epoxidation of a Variety of Olefins

The same experimental set-up described in Example 2 was employed with 5-ethyl-2-norbornene, bicyclo[2.2.2]oct-2-ene, and 5-norbornene-2-carboxaldehyde as the olefin feeds. Catalyst B was used for each of these runs. Reaction parameters and results are set forth in Table 5.

Table 5

| Selective Epoxidation of Other Bicyclic Olefins | | | | | |
|---|---|---|---|---|---|
| | Reaction Parameters | | | Bicyclic Olefin Conversion, % | Product Selectivity, %** |
| Bicyclic Olefin | Reaction Temp., °C | GHSV, $hr^{-1}$ | Gas Feed ($He/Olefin/O_2$)* | | |
| 5-ethyl-2-norbornene | 225 | 700 | 0.47:0.03:0.50 | 4 | 66 |
| | 225 | 700 | 0.43:0.07:0.50 | 1 | 78 |
| bicyclo[2.2.2] oct-2-ene | 210 | 460 | 0.47:0.03:0.50 | 4 | 36 |
| | 210 | 360 | 0.59:0.08:0.33 | 2 | 39 |
| 5-norbornene-2-carboxaldehyde | 219 | 255 | 0.665:0.005:0.33 | 55 | 4 |

*Volumetric ratio of feed components

**Product selectivity is defined as the molar selectivity to bicyclic olefin epoxide based on the moles of bicyclic olefin converted. Carbon dioxide was the only other carbon-based product formed in the reaction.

These results demonstrate that bicyclic olefins other than norbornene can also be selectively oxidized to the corresponding mono-epoxide derivative according to the practice of the present invention. The above results further indicate the importance of determining the preferred source (and loading level) of catalyst promoter to employ for a given feed olefin. For example, while 5-norbornene-2-carboxaldehyde gave only 4% selectivity to the corresponding epoxide under the reaction conditions employed (balance $CO_2$), the epoxidation of norbornene under the same reaction conditions using a catalyst of similar composition gave norbornene oxide with a selectivity in the range of about 80-90%. The results presented in Table 3 suggest that variations of the nature and quantity of promoter employed should afford highly selective epoxidation of a wide range of bicyclic olefins such as those set forth above.

EXAMPLE 6 - Optional Use of Organic Halide Co-feed

The effect of organic halide co-feed on norbornene conversion and selectivity to norbornene oxide was investigated. Thus, the oxidation of norbornene was carried out using Catalyst J according to the procedure described in Example 2. In addition, organic halide (1,2-dichloroethane was used in these experiments) was added via a mass flow controller which delivered a gas stream containing 100 parts per million (ppm; by volume) of organic halide (in helium diluent) to the reactant feed stream. By proper selection of flow rate for the 100 ppm organic halide-containing feed as diluted into the helium/bicyclic olefin/oxygen feed stream, it was possible to very accurately and reproducibly control the level of organic halide in the process feed stream.

Table 6 illustrates the controlled and reversible effects of co-feeding an organic halide such as 1,2-dichloroethane to a reaction zone employed for the selective oxidation of norbornene to norbornene oxide. The data are presented in the order in which the experiments were conducted. The cumulative amount of time on stream is also shown in the Table in order to illustrate the effect of the length of catalyst exposure to each specific set of reaction conditions. All runs were carried out at 225°C and a GHSV of 270 $hr^{-1}$ with Catalyst J.

Table 6

| Effect of 1,2-Dichloroethane Co-feed | | |
|---|---|---|
| Cumulative Reaction Time, min. | % Norbornene Conversion | Selectivity to Norbornene Oxide, %* |
| 227** | 43.1 | 89.0 |
| 247 | 43.1 | 89.0 |
| 262*** | 40.5 | 89.5 |
| 285 | 40.2 | 89.5 |
| 304 | 40.4 | 90.0 |
| 360**** | 40.1 | 90.3 |
| 408***** | 44.2 | 89.5 |
| 432 | 44.2 | 88.6 |
| 461 | 43.5 | 88.1 |

*Selectivity to norbornene oxide determined the same as described in earlier Tables

**Reaction time from 0 minutes up to 227 minutes was used to establish a steady state of catalytic activity for the reaction.

***Introduction of 5 ppm of 1,2-dichloroethane to the feed stream commenced after catalyst had been on stream for 250 minutes.

****Level of 1,2-dichloroethane co-feed adjusted from 5 up to 10 ppm after catalyst had been on stream for 320 minutes

*****Introduction of 1,2-dichloroethane co-feed into the feed stream discontinued after catalyst had been on stream for 390 minutes

The results in Table 6 demonstrate that the addition of organic halide co-feed to the reaction mixture helps to stabilize the conversion level achieved by a given catalyst. Thus, the conversion of bicyclic olefin can be controlled by the addition of organic halide to the feed stream. The above results further demonstrate that the effect of organic halide on catalyst performance is reversible with respect to both feed olefin conversion and product selectivity. Moreover, the above results demonstrate that the addition of organic halide co-feed to the feed stream can enhance the selectivity to the desired epoxide product.

The examples have been provided merely to illustrate the practice of our invention and should not be read so as to limit the scope of our invention or the appended claims in any way. Reasonable variations and modifications, not departing from the essence and spirit of our invention, are contemplated to be within the scope of patent protection desired and sought.

**Claims**

1. A process for the selective epoxidation of (2.x.y] bicyclic olefins having the structure:

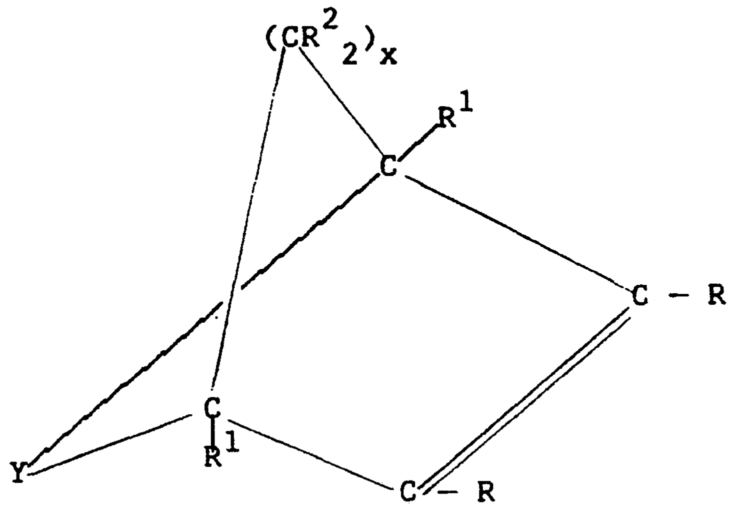

wherein each R is independently selected from the group consisting of:

a) hydrogen,

b) aryl and substituted aryl groups having in the range of 6 up to 20 carbon atoms,

c) tertiary alkyl groups of the formula:

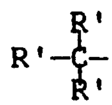 ,

where each R′ is independently:

a $C_1$-$C_6$ alkyl or substituted alkyl group,

a $C_6$-$C_{20}$ aryl or substituted aryl group,

$CR''_3(CR''_2)_n-\overset{O}{\overset{\|}{C}}-$,

$CR''_3(CR''_2)_n-\overset{O}{\overset{\|}{C}}-O-$, or

$R''O-\overset{O}{\overset{\|}{C}}-$,

where R″ is H, $C_1$-$C_{10}$ alkyl or substituted alkyl, an aryl or substituted aryl group having 6 up to 20 carbon atoms, and n is a whole number from 0-12;

d) $CR''_3-(CR''_2)_x-O-$, where x is a whole number from 1-12;

e) $CR''_3(CR''_2)_n-\overset{O}{\overset{\|}{C}}-$;

f) $CR''_3(CR''_2)_n-\overset{O}{\overset{\|}{C}}-O-$;

g) $R''O-\overset{O}{\overset{\|}{C}}-$;

h) $R''_2N-\overset{O}{\overset{\|}{C}}-$; i) $R''_2N-$;

j) $R''S-$;

k) $CR''_2=CR''-(CR''=CR'')_y$, where y is an integer from 0-20; and

l) 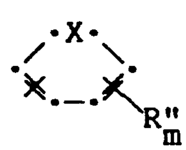 ,

where X is O, S or NR″; and m is an integer from 0-3,

with the proviso that the R groups have no allylic hydrogens;

each $R^1$ of said bicyclic olefin is H or a $C_1$-$C_{20}$ hydrocarbyl or substituted hydrocarbyl moiety;
each $R^2$ of said bicyclic olefin is H or a $C_1$-$C_{20}$ hydrocarbyl or substituted hydrocarbyl moiety;
x is a whole number which can vary from 1 up to 4;
Y is either an alkylene moiety of the structure:
$-(CR'_2)_y-$
wherein the R substituents are as defined above and each R is selected independently of the other, and y is a whole number which can vary from 1 up to 4, or Y is an alkenylene moiety of the structure:
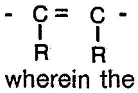
wherein the R′ substituents are as defined above and each R′ is selected independently of the other, and y for such alkenylene moiety is deemed to be 2;
said process comprising contacting said olefin with a sufficient quantity of an oxygen-containing gas so as to maintain the molar ratio of olefin to oxygen in the range of 0.01 up to 20, in the presence of a silver-containing catalyst at a pressure in the range of 0.1 up to 100 atmospheres, at a temperature in the range of 75 up to 325° C for a time sufficient to obtain olefin conversions in the range of 0.1 up to 75%.

2. A process in accordance with Claim 1 wherein said silver catalyst is a supported silver catalyst comprising in the range of 0.1 up to 50 weight % elemental silver.

3. A process in accordance with Claim 2 wherein said supported silver catalyst is supported on an inorganic support having a surface area no greater than about 50 $m^2/g$.

4. A process in accordance with Claim 3 wherein said inorganic support is selected from the group consisting of
silica,
alumina,
silica-alumina,
titanium oxide,
lanthanum oxide,
magnesium oxide,
boron nitride,
boron carbide,
silicon nitride,
silicon carbide,
zinc oxide,
tin oxide,
iron oxide,
calcium oxide,
barium oxide,
strontium oxide,
zirconium oxide,
carbon,
boron phosphate,
zirconium phosphate,
and mixtures of any two or more thereof.

5. A process in accordance with Claim 2 wherein said silver catalyst further comprises in the range of 0.001 up to 10 weight %, based on the total weight of catalyst, including support, of at least one promoter selected from the group consisting of:
the salts of alkali metals,
the oxides of alkali metals,
the salts of alkaline earth metals,
the oxides of alkaline earth metals,
organic halides,
inorganic halides,
acid halides, and
elemental halogens,
as well as mixtures of any two or more thereof.

6. A process in accordance with Claim 5 wherein said promoter is a halide salt of an alkali metal or a nitrate salt of an alkali metal or a mixture of any two or more thereof.

7. A process in accordance with Claim 1 wherein said olefin is selected from the group consisting of:
norbornene,

norbornadiene,
bicyclo[2.2.2]oct-2-ene,
5-ethyl-2-norbornene,
5-norbornene-2-carboxaldehyde,
as well as mixtures of any two or more thereof.

8. A process in accordance with Claim 7 wherein said silver catalyst comprises:
in the range of about 1 up to 30 weight % silver,
in the range of about 0.001 up to 10 weight percent of an alkali metal halide, an alkali metal nitrate, or a mixture of any two or more thereof, and
an alumina support having a surface area of less than about 10 $m^2/g$;
wherein said weight percentages are based on the total weight of catalyst.

9. A process in accordance with Claim 7 wherein said silver catalyst comprises:
in the range of about 2 up to 20 weight percent silver,
in the range of 0.01 up to 2 weight percent of an alkali metal halide, an alkali metal nitrate, or a mixture of any two or more thereof, and
an alumina support having a surface area of less than about 1 $m^2/g$;
wherein said weight percentages are based on the total weight of catalyst.

10. A process in accordance with Claim 9 wherein said alkali metal halide is selected from the group consisting of cesium chloride and cesium bromide, and said alkali metal nitrate is selected from the group consisting of cesium nitrate and rubidium nitrate.

11. A process in accordance with Claim 8 wherein said contacting is carried out at a temperature in the range of about 125 up to 275 °C, at a pressure in the range of about 1 up to 30 atmospheres for a time sufficient to obtain olefin conversions in the range of about 2 up to 75%.

12. A process in accordance with Claim 9 wherein said contacting is carried out at a temperature in the range of about 125 up to 275 °C, at a pressure in the range of about 1 up to 30 atmospheres for a time sufficient to obtain olefin conversions in the range of about 2 up to 30%.

13. A process in accordance with Claim 1 wherein said oxygen-containing gas is selected from the group consisting of:
air,
inert gas diluted air,
inert gas diluted oxygen,
oxygen-enriched air, and substantially purified oxygen.

European Patent Office

# EUROPEAN SEARCH REPORT

Application Number

EP 90 42 0130

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF THE AMERICAN CHEMICAL SOCIETY, vol. 110, no. 25, 7th December 1988, pages 8540-8541, American Chemical Society, Washington, US; J.T. ROBERTS et al.: "Epoxidation of olefins on silver: conversion of norbornene to norbornene oxide by atomic oxygen on Ag(110)"<br>* The whole article, particularly page 8541, right-hand column, last paragraph of the text *<br>--- | 1-13 | C 07 D 301/10<br>C 07 D 303/04<br>C 07 D 303/32 |
| P,X | EP-A-0 326 392 (EASTMAN KODAK)<br>* The whole document *<br>----- | 1-13 | |
| | | | **TECHNICAL FIELDS SEARCHED (Int. Cl.5)** |
| | | | C 07 D 301/00<br>C 07 D 303/00 |

The present search report has been drawn up for all claims

| Place of search | Date of completion of the search | Examiner |
|---|---|---|
| THE HAGUE | 26-06-1990 | ALLARD M.S. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document